Europäisches Patentamt

European Patent Office

Office européen des brevets

(19)

(11) Publication number: 0 453 001 A1

# EUROPEAN PATENT APPLICATION

(21) Application number: 91200173.2

(51) Int. Cl.5: A61K 9/24, A61K 9/54

(22) Date of filing: 29.01.91

(30) Priority: 17.04.90 IT 2005490

(43) Date of publication of application:
23.10.91 Bulletin 91/43

(84) Designated Contracting States:
AT BE CH DE DK ES FR GB GR IT LI LU NL SE

(71) Applicant: GIULIANI S.p.A.
Via P. Palagi, 2
I-20129 Milano(IT)

(72) Inventor: Calanchi, Massimo
Via Calatafimi, 12
I-20052 Monza(IT)

Inventor: Zema, Marco
Via Verga, 10
I-22100 Como(IT)
Inventor: Brunetti, Gabriele
Viale dei Mille, 27
I-20100 Milan(IT)
Inventor: Giorgetti, Enzo
Via Palmanova, 191
I-20100 Milan(IT)

(74) Representative: Appoloni, Romano et al
Ing. Barzanò & Zanardo S.p.A. Via
Borgonuovo 10
I-20121 Milano(IT)

(54) Pharmaceutical composition for the targeted controlled release of an active principle within the intestine, and particularly within the colon.

(57) The invention provides pharmaceutical compositions with the property of targeted controlled release of active principles which act pharmacologically within the intestine and in particular within the colon and the terminal portion of the ileum.

To attain this object the active principle is prepared in multiparticle multidose form and is covered with at least two membranes, one of pH-dependent solubility and the other insoluble but permeable to the intestinal fluids.

While the covered active principle remains in the stomach and in the initial intestinal portion, ie while the pH is less than 5.5, it is not released.

Only when it reaches an environment of higher pH (small intestine or colon) does the pH-dependent membrane dissolve to commence release of the active principle.

From this moment the second membrane, which is pH-independent but permeable to the intestinal fluids, acts to slow down and control the dissolution of the medicament within the small intestine-colon tract.

This invention relates to pharmaceutical compositions for obtaining targeted controlled release of active principles required to act pharmacologically within the intestine, and particularly within the terminal portion of the ileum and the colon.

USA patent 4,503,030 relates to osmotic release tablets consisting of a core containing the medicament covered with a pH-dependent semipermeable membrane containing a hole connecting the core to the outside. The tablet remains integral in the stomach, release occurring through the hole in the membrane, whereas in the intestine the membrane disintegrates completely.

USA patent 4,432,966 describes the preparation of tablets which disintegrate in the colon. This is obtained by covering the tablet core with two layers, the first consisting of a pH-independent polymer and microcrystalline cellulose, and the second a pH-dependent polymer.

The microcrystalline cellulose together with the pH-independent polymer ensure disintegration of the tablet within the colon, as the microcrystalline cellulose is digested by specific enzymes and bacteria present in the colon.

A problem relating to the use of such known compositions is the possible occurrence of mucosa irritation phenomena due to a high local medicament concentration.

An object of the present invention is to solve this problem for determined classes of active principle.

A requirement of the present invention is that the core or membrane of the pharmaceutical composition must not disintegrate within the colon, but instead the membrane must remain whole in order to slow down the dissolution of the medicament, and consequently prolong its action along the intestine, the colon and the terminal portion of the ileum, with a substantial improvement in the active principle absorption.

The present invention is aimed at active principles which exhibit their activity within the intestine, and particularly in the colon. As is well known, certain diseases of the intestine, and particularly of the colon, are cured by active principles with topical action. It is therefore important for such active principles to be transported intact into the site in which their pharmacological action is to take effect.

According to the present invention it is essential that the release of the active principle and its contact with the mucosa takes place along the entire colon. It is therefore necessary to slow down the release so that the effect is prolonged in time and acts on all parts subject to the disturbance and not only on the initial tract.

The aforesaid objects are attained according to the present invention by a pharmaceutical composition for the targeted controlled release of an active principle within the intestine, and in particular within the terminal portion of the ileum and the colon, characterised in that said active principle is chosen from the following: antiinflammatory-antiphlogistic agents of local and general action, such as 5-aminosalicylic acid (or mesalazine) and analogous salicylic derivatives, peppermint oil, corticosteroids of the kind directed to the local treatment of chronic intestinal diseases of inflammatory and irritative type; said active principle being formulated in multiparticle multidose form, each particle thereof being covered with at least two membranes, one of which is soluble at a pH equal to or greater than 5.5 and the other of said membranes being insoluble at said pH but permeable to the intestinal fluids.

The present invention is applicable to multiparticle multidose forms, ie to active principles prepared in particles in the form for example of crystals, granules, pellets or very small-dimension tablets (also known as minitablets), which are covered as described hereinafter. These covered active principles are then formulated as capsules, single-dose sachets, rapid disintegration tablets or other pharmaceutical forms suitable for oral administration.

Thus according to the invention it has been found that by applying separately to the active principle particles a membrane of pH-dependent solubility and a membrane which is insoluble in but permeable to the intestinal fluids, the dissolution of the active principle is slowed down so that it is released slowly and can thus exercise its action along the entire colon. For example, if an active principle is covered with a membrane which dissolves at a pH greater than 6 there is very slow release in buffer solutions up to pH 6.2 (first three hours). However, when the pH passes to 7.2 the composition rapidly dissolves.

By applying over the first membrane another membrane which is insoluble in the intestinal fluids but permeable to them, the release of the medicament is slowed down and the effect prolonged for a further 3 hours. According to the invention the same result is obtained by applying firstly the delay membrane and then the pH-dependent membrane, whereas if the constituent substances of the two membranes are mixed together the delay effect is not obtained and instead the release is very similar to that obtained by applying only the pH-dependent polymer.

The present invention therefore provides for applying in succession, in either order, a membrane soluble at a given pH and a membrane which is insoluble in but permeable to the intestinal fluids.

In this manner the release of the active principle can be targeted towards a certain tract of the intestine (colon) and also be prolonged to thus make it effective along the entire remaining intestinal tract.

The dimensions of the individual units of the multidose forms, ie of the individual crystals, granules, pellets or minitablets, vary between 0.1 and 3.5 mm, it being in any event advisable not to exceed 5 mm. In this respect, the smaller the individual units the more widespread is their distribution within the gastrointestinal tract, and in addition whereas units exceeding 5 mm are retained in the full stomach, units of less than 5 mm pass through the stomach more rapidly and in a manner similar to liquids. This phenomenon is described for example in the article by S.S. Davis entitled "The design and evaluation of the controlled release systems for the gastrointestinal tract" published in the "Journal of Controlled Release", 2 (1985) 27-38.

As the active principles are often in the form of fine powders, they are generally granulated using known wet or dry (compacting) methods, to obtain the desired particle size.

It should be noted that in the following description, that which refers to active principles in granular form is also valid for the other multiparticle multidose forms, ie crystals, pellets, minitablets etc.

The granulated active principle is placed in the container of a Glatt UNI fluidized bed provided with a Wurster insert and, by spraying with a suitable atomizer, is covered with a pH-dependent polymer dissolved in an organic solvent or in a mixture of organic solvents or in a mixture of organic solvent or solvents and water, or in an aqueous solution, dispersion or emulsion.

It is also convenient to add plasticizers and adjuvants. Polymer types for forming the pH-dependent membrane include by way of non-limiting example: copolymers of methacrylic acid (Eudragit L, Eudragit S), cellulose acetate phthalate, hydroxypropyl methylcellulose phthalate, polyvinylacetate phthalate, shellac, hydroxypropylmethylcellulose acetate succinate, carboxymethylcellulose, cellulose acetate trimellitate, copolymers of maleic acid and phthalic acid derivatives.

Plasticizers include by way of non-limiting example: polyethylene glycol, dibutyl phthalate, diethyl phthalate, citric acid esters. Other adjuvants include: talc, silicon dioxide, titanium dioxide, magnesium stearate.

The covered granules are dried with hot air (about 50°C) for about 30 minutes.

A second pH-independent membrane permeable to intestinal fluids is then applied to the granules covered with the pH-dependent membrane.

Organic or aqueous solutions or aqueous dispersions/emulsions can again be used, and it is again convenient to add plasticizers and adjuvants of the aforesaid type.

Polymer types for forming the pH-independent membrane include by way of non-limiting example: copolymers of methacrylic esters (Eudragit RS/RL/NE), ethylcellulose, polyethylene and polysiloxanes, either alone or mixed with each other or with other pH-independent polymers soluble in water, such as: hydroxypropylmethylcellulose, hydroxypropylcellulose, hydroxyethylcellulose, methylcellulose, polyvinylpyrrolidone.

The granules covered with the membranes are dried with hot air (about 50°C) for about 30 minutes.

As already stated, the two membranes can be applied in the reverse order to that described.

Some non-limiting examples of the practical implementation of the invention are described hereinafter.

EXAMPLE 1

800 g of 5-aminosalicylic acid granulated with hydroxypropylmethyl cellulose and having a particle size of between 710 and 1300 μm are placed in the container of a Glatt UNI fluidized bed provided with a Wurster insert.

This granulate is covered with a first membrane of Eudragit S, by spraying with a suitable atomizer a suspension having the following composition: 468 g of methylene chloride, 156 g of isopropyl alcohol, 55.6 g of Eudragit S, 5.5 g of dibutylphthalate and 28 g of talc.

The covered granules were dried with hot air (about 50°C) for 30 minutes and their release was then determined with the USP apparatus (bladed stirrer), using the following succession of artificial juices: 2 hours in 0.1 N HCl; 1 hour in pH 6.2 buffer and the subsequent hours in pH 7.2 buffer.

The following results were obtained:

| time (hours) | 1 | 2 | 3 | 4 | 5 |
|---|---|---|---|---|---|
| release (%) | 6 | 11 | 12 | 61 | 96 |

A second membrane of ethylcellulose was then applied to 700 g of these granules covered with Eudragit S, by spraying the following solution: 199 g of methylene chloride, 44 g of ethyl alcohol, 4.3 g of ethyl cellulose, 8.6 g of hydroxypropylmethylcellulose and 1.5 g of diacetylated monoglycerides, and finally drying with air at 50°C for about 30 minutes.

The granules covered with the two membranes were again tested in the aforedescribed manner, obtaining the following results:

| time (hours) | 1 | 2 | 3 | 4 | 5 | 6 | 8 |
|---|---|---|---|---|---|---|---|
| release (%) | 4 | 9 | 9 | 26 | 51 | 74 | 95 |

EXAMPLE 2

700 g of 5-aminosalicylic acid granulated with hydroxypropylmethyl cellulose and having a particle size of between 710 and 1300 μm are placed in the container of a Glatt UNI fluidized bed provided with a Wurster insert.

This granulate is covered with a first membrane of ethylcellulose/hydroxypropylmethylcellulose, by spraying with a suitable atomizer a solution having the following composition: 200 g of methylene chloride, 45 g of ethyl alcohol, 6.4 g of ethylcellulose, 6.4 g of hydroxypropylmethylcellulose and 1.4 g of diacetylated monoglycerides.

The covered granules were dried with hot air (about 50°C) for 30 minutes and their release was then determined with the USP apparatus (bladed stirrer), using the following succession of artificial juices: 2 hours in 0.1 N HCl, 1 hour in pH 6.2 buffer and the subsequent hours in pH 7.2 buffer.

The following results were obtained:

| time (hours) | 1 | 2 | 3 | 4 | 5 |
|---|---|---|---|---|---|
| release (%) | 31 | 56 | 74 | 86 | 100 |

A second membrane of Eudragit S was then applied to these covered granules by spraying the following suspension:134 g of methylene chloride, 65 g of isopropyl alcohol, 23 g of Eudragit S, 2.3 g of dibutyl phthalate and 11.5 g of talc, and finally drying with air at 50°C for about 30 minutes.

The granules covered with the two membranes were again tested in the aforedescribed manner, obtaining the following results:

| time (hours) | 1 | 2 | 3 | 4 | 5 | 6 | 8 |
|---|---|---|---|---|---|---|---|
| release (%) | 4 | 7 | 11 | 36 | 59 | 75 | 100 |

EXAMPLE 3 (comparison)

800 g of 5-aminosalicylic acid granulated with hydroxypropylmethyl cellulose and having a particle size of between 710 and 1300 μm are placed in the container of a Glatt UNI fluidized bed provided with a Wurster insert.

This granulate is covered with a membrane of ethylcellulose/hydroxypropylmethylcellulose/Eudragit S, by spraying with a suitable atomizer a suspension having the following composition: 836 g of methylene chloride, 418 g of isopropyl alcohol, 5.8 g of ethylcellulose, 11.8 g of hydroxypropylmethylcellulose, 58.7 g of Eudragit S, 3.7 g of dibutylphthalate and 29 g of talc.

The covered granules were dried with hot air (about 45°C) for 30 minutes an their release was then determined with the USP apparatus (bladed stirrer), using the following succession of artificial juices: 2 hours in 0.1 N HCl, 1 hour in pH 6.2 buffer and the subsequent hours in pH 7.2 buffer.

The following results were obtained:

| time (hours) | 1 | 2 | 3 | 4 | 5 | 6 |
|---|---|---|---|---|---|---|
| release (%) | 3 | 5 | 6 | 54 | 89 | 100 |

A comparison of the results of the active principle release obtained with the pharmaceutical compositions of Examples 1 and 2 with the results obtained with the composition of the comparison Example 3 demonstrates the advantages of the invention compared with the case in which a single membrane covering is provided. The applicant also conducted clinical tests with the pharmaceutical composition of Example 2, the methods used and the results obtained being described briefly below.

A study was conducted on the release of the mesalazine contained in the composition of said Example 2 within the terminal portion of the ileum and within the colon both of healthy volunteers and of patients suffering from ulcerative colitis and Crohn's disease, using radiological methods.

To monitor the granule transit and dispersion within the intestinal tract each subject received one capsule of a test preparation containing 600 mg of iodamide granules, with a repeat test after 7 days.

The location of the granules at the various levels and their dispersion were visually displayed by taking radiographs of the abdominal region, these being repeated every 2 hours until 10 hours from administration of the composition, and then after 12 or 24 hours if granules were still visible.

The radiographs obtained were then examined in a blind test, the following parameters being considered in radiographically evaluating the behaviour of the granules of said composition:

1) homogeneous presence of granules in the jejunal ansa and ileal ansa;
2) presence of granules in the distal ileum;
3) presence of granules in the proximal colon;
4) time for numerical reduction in the sense of an appreciable reduction in the granule quantity, to the extent of about 25% of the overall initial quantity;
5) reduction in opacity in the sense of a significant attenuation in the radio-opacity of the granules;
6) time for maximum numerical reduction in the sense of a considerable reduction in the granule quantity, of greater than 75%.

The results obtained showed that the pharmaceutical composition of Example 2 of the present invention is able to release the active principle in accordance with the initially stated requirements, with radiologically determined dissolution taking place mainly in the proximal colon.

## Claims

1. A pharmaceutical composition for the targeted controlled release of an active principle within the intestine, and in particular within the terminal portion of the ileum and the colon, characterised in that said active principle is chosen from the following: antiinflammatory-antiphlogistic agents of local and general action, such as 5-aminosalicylic acid (or mesalazine) and analogous salicylic derivatives, peppermint oil, corticosteroids of the kind directed to the local treatment of chronic intestinal diseases of inflammatory and irritative type; said active principle being formulated in multiparticle multidose form, each particle thereof being covered with at least two membranes, one of which is soluble at a pH equal to or greater than 5.5, the other of said membranes being insoluble at said pH but permeable to the intestinal fluids.

2. A composition as claimed in claim 1, characterised in that said active principle is covered with a first membrane soluble at said pH values, this first membrane being covered with a second membrane insoluble at said pH values but permeable to the intestinal fluids.

3. A composition as claimed in claim 1, characterised in that the active principle is covered with a first membrane insoluble at said pH values but permeable, and being covered with a second membrane soluble at said pH values in natural or artificial fluids.

4. A composition as claimed in claim 1, characterised in that said active principle in multiparticle multidose form is in the form of crystals, granules, pellets or minitablets having a particle size distribution of between 0.1 and 3.5 mm.

5. A composition as claimed in claim 1, characterised in that said membrane soluble at a pH equal to or greater than 5.5 consists of one or more of the following polymers: copolymers of methacrylic acid (Eudragit L, Eudragit S), cellulose acetate phthalate, hydroxypropylmethylcellulose phthalate, polyvinyl acetate phthalate, shellac, hydroxypropylmethylcellulose acetate succinate, carboxymethylcellulose, cellulose acetate trimellitate, copolymers of maleic acid and phthalic acid derivatives.

6. A composition as claimed in one or more of the preceding claims, characterised in that the membrane insoluble at said pH values but permeable to the intestinal fluids consists of copolymers of methacrylic esters (Eudragit RS/RL/NE), ethylcellulose, polyethylene and polysiloxanes, either alone or mixed with each other or with other pH-independent polymers soluble in water, such as hydroxypropylmethylcellulose, hydroxypropylcellulose, hydroxyethylcellulose, methylcellulose, polyvinylpyrrolidone.

7. A composition as claimed in claim 5, characterised in that said membrane (pH-dependent) consists of Eudragit S.

8. A composition as claimed in claim 6, characterised in that said membrane (pH-independent) is ethylcellulose, or ethylcellulose mixed with hydroxypropylmethylcellulose in a ratio varying from 1:3 to 3:1.

9. A composition as claimed in claim 1, characterised in that said membranes comprise further components such as plasticizers, preferably consisting of polyethylene glycol, dibutyl phthalate, diethyl phthalate, or citric acid esters.

10. A composition as claimed in claim 1, characterised in that said membranes also comprise adjuvants, preferably consisting of talc, silicon dioxide, titanium dioxide or magnesium stearate.

11. A composition as claimed in claim 1, characterised in that said active principle is 5-aminosalicylic acid (or mesalazine).

12. A composition as claimed in claim 1, characterised in that said active principle is peppermint oil.

European
Patent Office

**EUROPEAN SEARCH
REPORT**

Application Number

**EP 91 20 0173**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 239 361 (KINAFORM TECHNOLOGY,INC.)<br>* the whole document *<br>– – – | 1,3-6,<br>8-11 | A 61 K 9/24<br>A 61 K 9/54 |
| X | EP-A-0 148 811 (LEJUS MEDICAL AKTIEBOLAG)<br>* the whole document *<br>– – – | 1,3-11 | |
| X | EP-A-0 212 745 (THE PROCTER AND GAMBLE COM-<br>PANY)<br>* the whole document *<br>– – – | 1-2,4-7,<br>9-11 | |
| X | WO-A-8 503 437 (A/S ALFRED BENZON)<br>* the whole document *<br>– – – | 1,3-11 | |
| A,D | GB-A-2 066 070 (ROUSSEL-UCLAF)<br>* the whole document & US-A-4432966 *<br>– – – | 1,3-11 | |
| A | GB-A-2 134 785 (LEO PHARMACEUTICAL PRODUCTS<br>LTD. A/S)<br>* the whole document *<br>– – – – – | 1-12 | |

TECHNICAL FIELDS
SEARCHED (Int. Cl.5)

A 61 K

The present search report has been drawn up for all claims

| Place of search | Date of completion of search | Examiner |
|---|---|---|
| Berlin | 21 June 91 | SIATOU E |

CATEGORY OF CITED DOCUMENTS

X : particularly relevant if taken alone
Y : particularly relevant if combined with another
document of the same category
A : technological background
O : non-written disclosure
P : intermediate document
T : theory or principle underlying the invention

E : earlier patent document, but published on, or after
the filing date
D : document cited in the application
L : document cited for other reasons

& : member of the same patent family, corresponding
document